# EUROPEAN PATENT SPECIFICATION

(11) **EP 2 768 576 B1**
(45) Date of publication and mention of the grant of the patent: **25.07.2018**
(21) Application number: 12842438.9
(22) Date of filing: 19.10.2012
(51) Int. Cl.: A61N 1/04

(54) **ELECTRODE APPARATUS WITH INTERFITTING ELEMENTS AND A METHOD FOR ITS MANUFACTURE INCLUDING STAMPING**
ELEKTRODENVORRICHTUNG MIT INEINANDERPASSENDEN ELEMENTEN UND EIN VERFAHREN ZU DEREN HERSTELLUNG UMFASSEND STANZUNG
APPAREIL À ÉLECTRODES AVEC DES ÉLÉMENTS INTERCALANTS ET UN PROCÉDÉ POUR SA FABRICATION COMPRENANT DE L'ESTAMPAGE

(30) Priority: 19.10.2011 US 201161627863 P
(43) Date of publication of application: 27.08.2014
(73) Proprietor: HTK Enterprises, Inc., Richardson, TX 75081 (US)
(72) Inventor: BYARS, Gary L., Richardson, TX 75081 (US); TURNER, David O., Royce City, TX 75189 (US); PAYNE, Charles H., Frisco, TX 75034 (US)
(74) Representative: Uexküll & Stolberg
(86) International application number: PCT/US2012/061172
(87) International publication number: WO 2013/059706

(56) References cited:
- US-A- 4 899 754
- US-A1- 2002 183 685
- US-A1- 2005 021 118
- US-A1- 2006 264 806
- US-A1- 2007 016 268
- US-A1- 2007 260 171
- US-A1- 2007 293 918
- US-A1- 2010 191 316
- US-A1- 2011 016 710
- US-A1- 2011 175 271
- US-B1- 8 000 804
- US-B2- 7 970 481

## Description

### TECHNICAL FIELD

The disclosure relates generally to a method of manufacturing electrode arrays and, more particularly, to an electrode manufacturing process in which two conductors are simultaneously stamped, the two conductors cooperating to form a plurality of virtual electrode pairs.

### BACKGROUND

Electrode arrays are typically manufactured by a process in which the individual electrodes are created and then placed in an array configuration. This presents a number of problems and inefficiencies. For example, it is inefficient to individually manufacture each electrode in the array. Second, with the array resulting from the prior manufacturing methods, individual wires have to be connected to each electrode in such a manner as to create two circuits. Thus, in a 3x3 electrode array using individual electrodes, for example, nine individual wires are used to complete all required connections.

US 2010/0191316 A1 relates to an electrode set and stimulating device. As described, the electrode set for a stimulation device, such as a TENS or EMS stimulation device comprises at least a plurality of neighboring electrically active zones close to each other said active zones forming a succession of poles of alternating polarity or being grouped to form groups of poles of alternating polarity, wherein at least one of said active zone or group of zones has a lateral size (D2) of approximately 1 mm to 40 mm, wherein at least one of spacing (D1) between neighboring active zones or groups of zones is approximately of 1 mm to 40 mm, and said set further comprises contacting means connected to said active zones or groups of active zones.

### SUMMARY

An apparatus is provided for manufacturing an electrode node array device. Furthermore, the device and a method for making the device are disclosed. In one example, an electrode node array apparatus is provided. The apparatus includes a first physical electrode element having a first plurality of electrode nodes formed therein interconnected by a common first conductor. The apparatus also includes a second physical electrode element having a second plurality of electrode nodes formed therein interconnected by a common second conductor. The first and second conductors are arranged to interfit the first and second pluralities of electrode nodes to form an array of electrode nodes.

In another example, a method of making an electrode node array is provided. The method includes providing a conductive sheet and forming a plurality of raised protrusions in the conductive sheet to correspond to a plurality of electrode nodes. The method further includes stamping a first conductor from the conductive sheet, the first conductor having a first set of the plurality of the raised protrusions interconnected by a common first conductor element. The method further includes stamping a second conductor from the conductive sheet, the second conductor having a second set of the plurality of raised protrusions interconnected by a common second conductor element. The method further includes interfitting the first and second conductors so that the first and second sets of raised protrusions are configured in an array of electrode nodes.

In another example, an apparatus for manufacturing an electrode node array device is provided. The apparatus includes a receiving tray having defined therein a plurality of recesses to correspond with a plurality of electrode nodes of the array device. The apparatus also includes one or more vacuum passages having a first end connectable to a vacuum source and a second end terminating at a hole at a surface of the apparatus. The hole is operable to interact with at least a portion of an array device positioned on the apparatus to hold at least a portion of the array device against the apparatus.

One or more of the examples may provide some, none, or all of certain of the following advantages. One advantage is improved reliability by the elimination of numerous wired connections. Another advantage is significant reduction in material and labor costs resulting from reducing part count and elimination of several manufacturing process steps such as, cutting/stripping wire, stamping an electrode which consists of three separate parts which require assembly and assembling nine individual electrodes.

### BRIEF DESCRIPTION OF THE DRAWINGS

For a more complete understanding of this disclosure and its features, reference is now made to the following description, taken in conjunction with the accompanying drawings, in which:
FIGURE 1 is an illustration of a conductive sheet according to an example;
FIGURE 2 is an illustration of an array of raised protrusions on a conductive sheet according to an example;
FIGURE 3 is an illustration of a stamped conductive sheet providing two physical electrode elements according to an example;
FIGURE 4 is an illustration of a stamped conductive sheet with attached electrical leads according to an example;
FIGURE 5 is a perspective view of a stamped conductive sheet with attached electrical leads being coupled to two laminate insulator sheets according to an example;
FIGURE 6 is an electrode array device according to an embodiment;
FIGURE 7 is a perspective view illustrating an apparatus for manufacturing an electrode array device and a stamped conductive sheet providing two physical electrode elements, and further illustrating a step in the manufacturing process;
FIGURE 8 is a perspective view illustrating an apparatus for manufacturing an electrode array device and a first laminate sheet being applied to two physical electrode elements, and further illustrating a step in the manufacturing process;
FIGURE 9 is a perspective view illustrating an apparatus for manufacturing an electrode array device and a first laminate sheet having been applied to two physical electrode elements, and further illustrating a step in the manufacturing process according to an embodiment;
FIGURE 10 is a perspective view illustrating an apparatus for manufacturing an electrode array device and a first laminate sheet having been applied to two physical electrode elements, the device prepared for receiving a second laminate sheet, and further illustrating a step in the manufacturing process according to an embodiment;
FIGURE 11 is a perspective view illustrating an apparatus for manufacturing an electrode array device and a first laminate sheet having been applied to two physical electrode elements, and a portion of the first laminate sheet folded to cover electrical contacts, and further illustrating a step in the manufacturing process;
FIGURE 12 is a perspective view illustrating an apparatus for manufacturing an electrode array device and a second laminate sheet being applied to two physical electrode elements, and further illustrating a step in the manufacturing process;
FIGURE 13 is a perspective view illustrating an apparatus for manufacturing an electrode array device and a second laminate sheet having been applied to two physical electrode elements, and further illustrating a step in the manufacturing process;
FIGURE 14 is a perspective view illustrating an apparatus for manufacturing an electrode array device and the device removed from the apparatus, and further illustrating a step in the manufacturing process; and
FIGURE 15 is a perspective view illustrating a completed electrode array device according to an embodiment.

### DETAILED DESCRIPTION

An electrode array apparatus is provided as well as a method of manufacturing the electrodes. The apparatus has an array of electrode nodes or virtual electrode pairs. However, the apparatus only has two physical electrode elements. Therefore, the virtual array of nodes created by the pair of physical electrodes only requires one pair of electrical leads and only creates one circuit. It should be understood that in other alternative cases, more than two physical electrode elements may be incorporated, while providing an array of virtual electrode nodes in a number that is greater than the number of physical electrode elements. For example, four physical electrode elements may provide eighteen virtual electrode nodes by incorporating the concepts described herein.

The invention is also directed toward a method of manufacturing the virtual electrode node array. The method is described after the description of an example array device.

In at least one case, a first physical electrode comprises a single, interconnected conductor having a first plurality of electrode nodes. The electrode nodes are interconnected by a common conductor element. The electrode nodes have a surface area larger than the interconnecting portion between any two of the electrode nodes. A second physical electrode is similarly constructed and comprises a second plurality of electrode nodes. The first and second plurality of electrode nodes interfit to form an array of electrode nodes.

The first and second conductors may be formed by a single metal stamping step which cuts the respective conductors out of a blank metal sheet. This is accomplished after a step of indenting, or extruding, portions of the sheet that will correspond to the electrode nodes. Preferably, the interconnections and the interfit configuration provide uniform gaps between the respective pairs of electrode nodes.

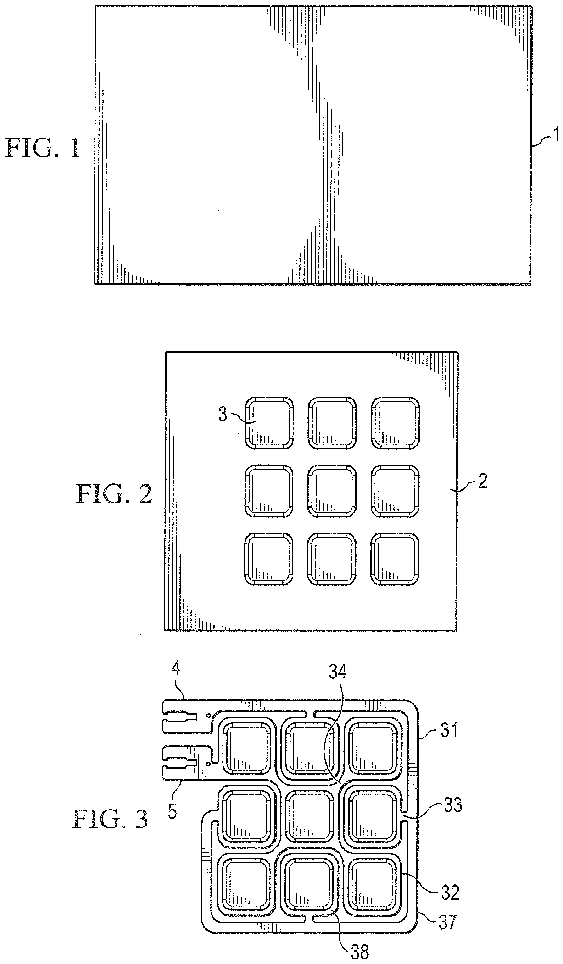

An example virtual electrode node array has a variety of components. As illustrated in FIGURE 1, for instance, a stock sheet 1 of a conductive material (e.g., stainless steel) is used as a base for the physical electrode elements. While stainless steel is used for example purposes, other conductive materials may be used as desired. In one example embodiment, the stock sheet is 0.3048 µm (0.012'') thick type 304 stainless steel. Preferably the thickness is in the range of about 0.2794 mm (0.011 inch) to about 0.3556 mm (0.014 inch). This is preferred for purposes of optimum metal flow.

FIGURE 2 illustrates a conductive sheet 2 that has been dimpled or extruded to provide a plurality of raised protrusions 3. Raised protrusions 3, at later stages of the manufacturing process, become the electrode nodes of the electrode array device.

FIGURE 3 illustrates the conductive sheet of FIGURE 2 after a stamping process. The process results in two physical electrode elements. A first physical electrode element 31 has a number of raised protrusions 3. Four raised protrusions 3 are shown, but it should be understood that the approach illustrated in FIGURE 3 may be used to provide additional raised protrusions. Protrusions 3 on element 31 are continuous with element 31 and are joined with an outer, surrounding portion 37 by way of bridge portions 33. The stamping process also results in a base 38 surrounding each of the protrusions 3. Protrusions 3 on element 31 are formed in a pattern according to which the protrusions alternate with an empty space in both of the two axes of an array of protrusions. This empty space is ultimately filled by protrusions 3 of second physical electrode element 32, which is described below.

Second physical electrode element 32 has five raised protrusions 3, although it should be understood that first and second physical electrode elements 31 and 32 may each be formed to provide more or fewer raised protrusions 3. The raised protrusions 3 of element 32 are formed in a pattern according to which the protrusions alternate with an empty space in both of the two axes of an array of protrusions. This empty space is ultimately filled by protrusions 3 of first physical electrode element 32, which is described above. Protrusions 3 of element 32 are each joined to at least one neighboring protrusion 3 of element 32 by a bridge portion 34.

First and second physical electrode elements 31 and 32 are also stamped to form first and second electrical lead tabs 4 and 5. Electrical lead tabs 4 and 5 are illustrated as being patterned to receive particular electrical leads. However, it should be understood that the electrical lead tabs may have different shapes and configurations to accept different types of electrical leads. Alternatively, the tabs may be simple extensions that accommodate soldered, crimped or other connection methods. Alternatively, no lead tabs are formed and the electrical leads are coupled directly to the respective first and second physical electrode elements 31 and 32.

The first and second physical electrode elements thus formed provide first and second electrical circuits. These circuits form the basis for the completed electrode apparatus.

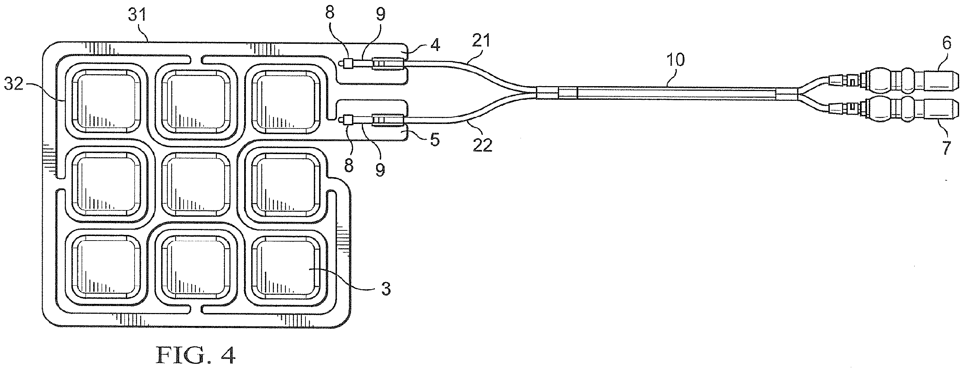

FIGURE 4 illustrates the stamped conductive sheet with an electrical lead attached. Electrical lead wire assembly 10 has touch-proof molded socket connectors 6, 7 for connecting to a power source (not shown). Lead wire assembly 10 has a pair of leads 21, 22 at an end opposite socket connectors 6, 7. Lead 21 is a positive lead and lead 22 is a negative lead. However, the polarity of the leads may be reversed as desired. Each lead 21, 22 has a weld tab 8 connected to the respective lead wire with a crimped connection 9. Lead wire assembly 10 may be connected to the electrode array by resistance welding the respective weld tabs 8 to the lead tabs 4 and 5.

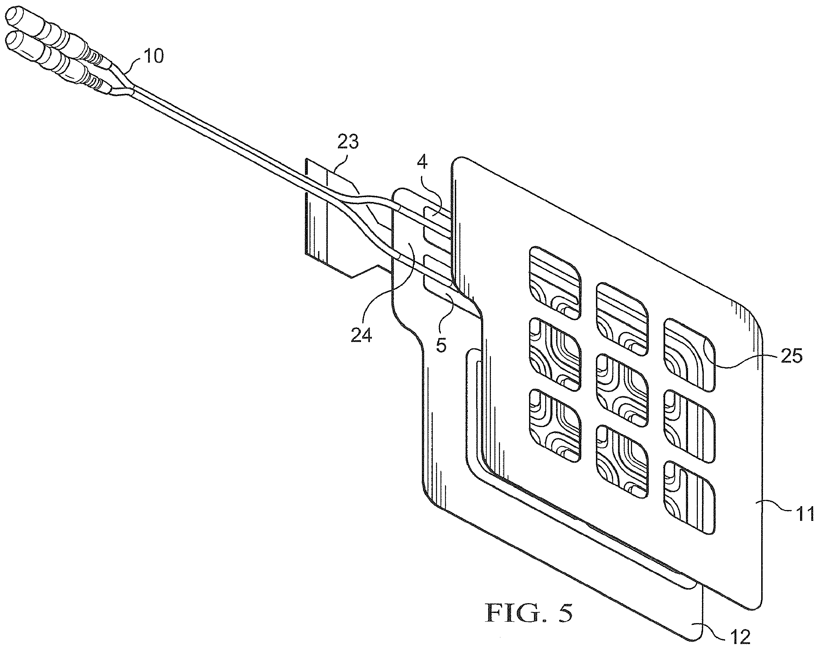

FIGURE 5 illustrates the stamped conductive plates with the lead wire assembly 10 connected and being sandwiched between two laminate sheets. A first laminate sheet 11 has a number of openings 25 for receiving raised protrusions 3. First laminate sheet 11 is applied to the stamped conductive sheet on the same side as the raised protrusions 3. A second laminate sheet 12 has a lead connector protective pad 23. Second laminate sheet 12 is applied to the side of the conductive sheet opposite the raised protrusions 3. Preferably, second laminate sheet 12 is applied first. Then the protective pad 23 is folded over between the electrical leads and disposed on area 24 of second laminate sheet 12 such that wing portions of the pad cover the respective electrical lead connections. Then, first laminate sheet 11 is applied. When first laminate sheet 11 is applied, protrusions 3 are disposed through openings 25 such that the backing of first laminate sheet 11 immediately surrounding respective openings 25 seats and seals against the bases 38 of the respective raised protrusions 3.

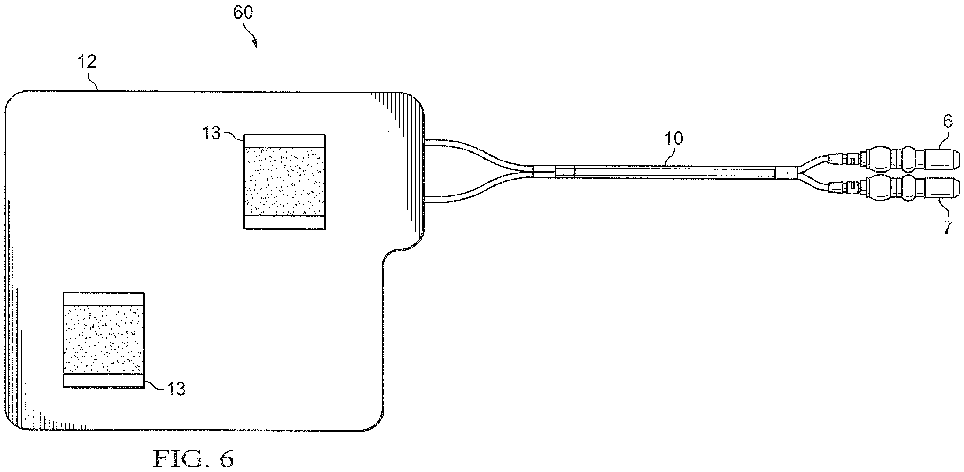

FIGURE 6 illustrates a completed electrode apparatus from the side opposite that shown generally in FIGURE 5. Apparatus 60 is preferably constructed according to the description herein. However, it should be understood that variations may be made in the manufacturing process and that the resulting apparatus may be either the same or somewhat different.

Apparatus 60 is illustrated from the view of second laminate sheet 12. A pair of Velcro® connectors 13 are provided for fastening to accessories (not shown) used in a neurostimulation procedure. It should be understood that the connectors may be greater or fewer in number, may have different placements, and may be of different types. The side viewed in FIGURE 6 may be referred to as the top side of the apparatus, while the opposite side would be applied to the target area (e.g., the skin of a human patient).

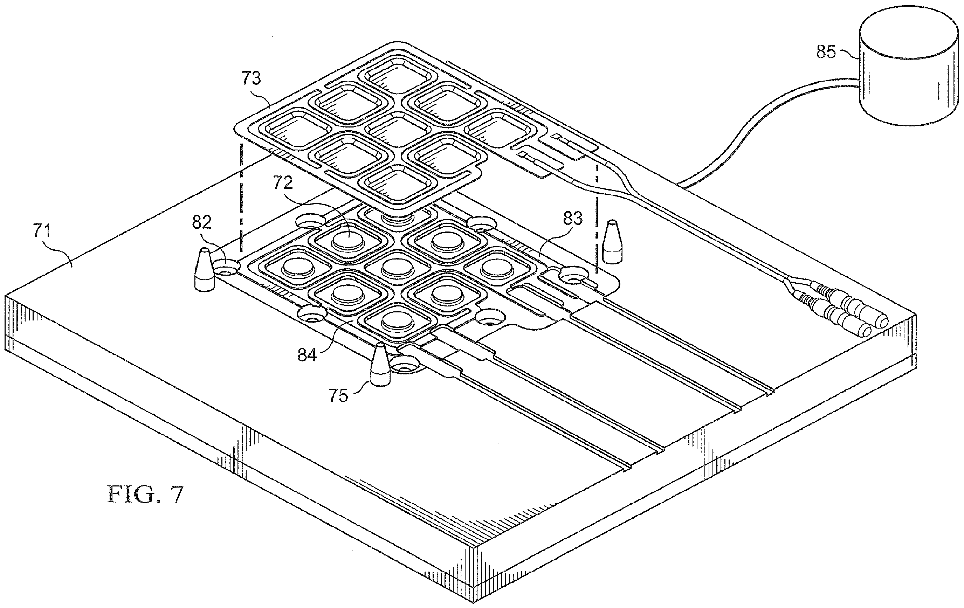

FIGURE 7 illustrates an apparatus for manufacturing a neurostimulation device according to an example. In at least one case, manufacturing apparatus 71 is a vacuum device which has formed therein a number of passageways (not expressly shown). The passageways are interconnected and/or connected to a vacuum pump 85. For simplicity, pump 85 is not shown in other figures illustrating the manufacturing process. One or more of the internal passageways terminates in a plurality of holes 82 at an upper surface of apparatus 71. One or more of the holes 82 are interconnected with vacuum paths 83. When a vacuum is applied to apparatus 71, air is drawn into holes 82 in a manner to create a suction on holes 82 and vacuum paths 83 in order to hold an object adjacent to one or more of the holes 82 and/or vacuum paths 83. Apparatus 71 also includes a plurality of suction cups 72. Suction cups 72 are operable to hold an object (preferably with a flat surface) against suctions cups 72. Apparatus 71 also includes a plurality of tapered alignment pins, which are operable to mate with holes in an object and align the object with respect to vacuum holes 82, vacuum paths 83 and suction cups 72. Apparatus 71 includes a receiving tray 84 for receiving an object. It is preferable that receiving tray 84 be configured to have a surface that matches with a surface of an object that will be placed in receiving tray 84 as described below. However, it should be understood that the configuration of the apparatus 71 illustrated in FIGURE 7 is an example only, and the various elements of apparatus 71 may be altered or otherwise reconfigured to mate with different objects, to provide different alignment features, and to provided different vacuum and suction characteristics. In at least one alternative case, the vacuum created by pump 85 also creates a vacuum by drawing air through one or more holes in suction cups 72.

FIGURE 7 also illustrates a stamped conductive plate 73 having connected electrical leads, such as may be formed according to one or more examples as already described. FIGURE 7 further illustrates a step in a process for manufacturing a neurostimulation device. The stamped conductive plate 73 is positioned within apparatus 71, such that the raised protrusions of the stamped conductive plate are facing downward to seat against suction cups 72. Preferably, there is a suction cup 72 for each of the raised protrusions and it is also preferable that each respective suction cup 72 be positioned generally in the center of the surface of the respective raised protrusion which stamped conductive plate 73 is positioned in receiving tray 84. However, alterations to this configuration may be made as desired. Once stamped conductive plate 73 is thus positioned, pressure is applied to stamped conductive plate 73 in order to assist suction cups 72 in creating a suction force to hold the respective surfaces of the raised protrusions.

In another step, vacuum pump 85 is activated to create a vacuum through one or more of the internal passageways in apparatus 71 and/or through one or more holes (in certain cases) in suction cups 72. Vacuum at multiple suction cups 72 provides a means to hold in a steady and precise alignment all 9 protrusions in stamped conductive place 73 in receiving tray 84. In at least one case, the vacuum pressure applied is in the range of 50.8 kPa (15" Hg) to 84.7 kPa (25" Hg). More preferably, the range is about 67.7 kPa (20" Hg) to 74.5 kPa (22" Hg). Even more preferably, the range is about 26 liters per minute.

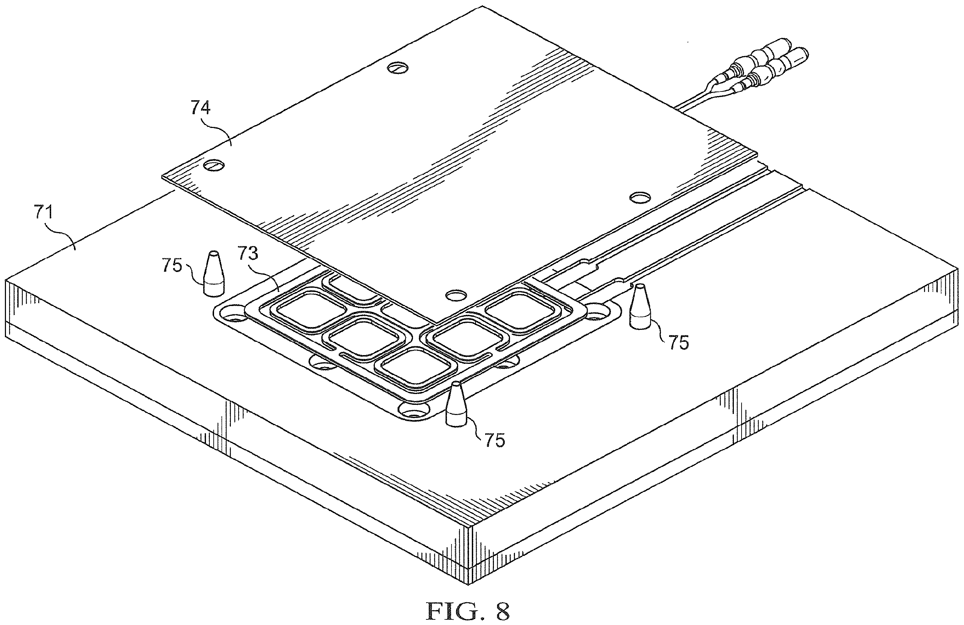

FIGURE 8 illustrates a stamped conductive plate 73 firmly seated in receiving tray 84 of apparatus 71 and held in place by vacuum at multiple suction cups 72. Also illustrated is the application of a first laminate 74 to stamped conductive plate 73. First laminate 74 preferably has a plurality of alignment holes to match the tapered alignment pins 75 of apparatus 71. First laminate 74 is positioned such that the alignment pins 75 are received through the laminate's alignment holes. This guides first laminate 75 into a position on top of what may be viewed as a back surface of stamped conductive plate 73. Preferably, first laminate 74 has an adhesive surface, which bonds to the surface of stamped conductive plate 73. Pressure may be applied to first laminate 74 to assist in the bonding process. Additionally, vacuum pressure provided as already described may further assist in the bonding process.

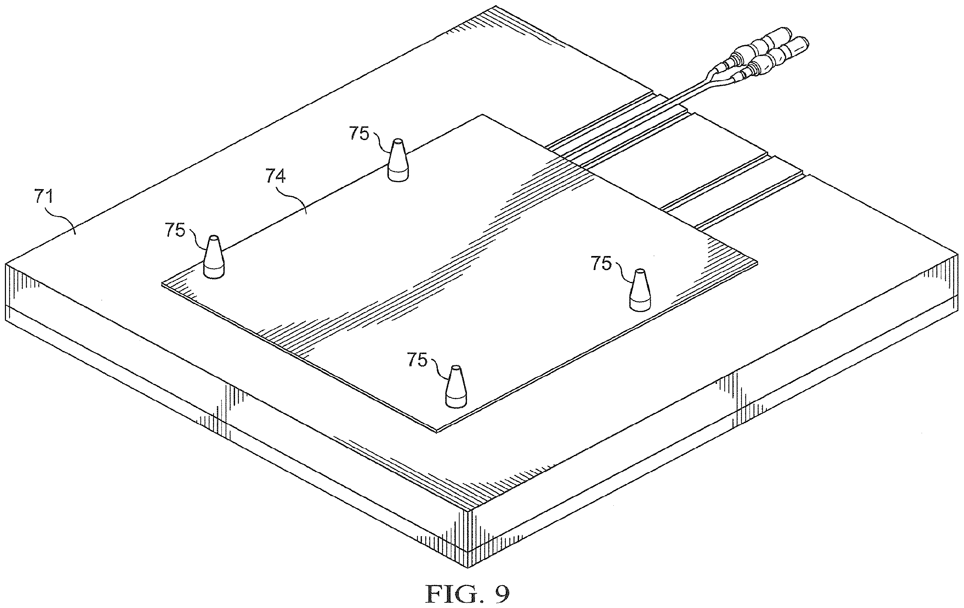

FIGURE 9 illustrates the first laminate firmly secured to the surface of the stamped conductive plate 73. If there is a liner on first laminate 74, such as a protective sheet for example, it may be removed. The vacuum pump 85 may now be turned off.

The partially-completed neurostimulation device may be removed from apparatus 71 and flipped over 180 degrees such that the raised protrusions are now facing upward and away from the receiving tray 84.

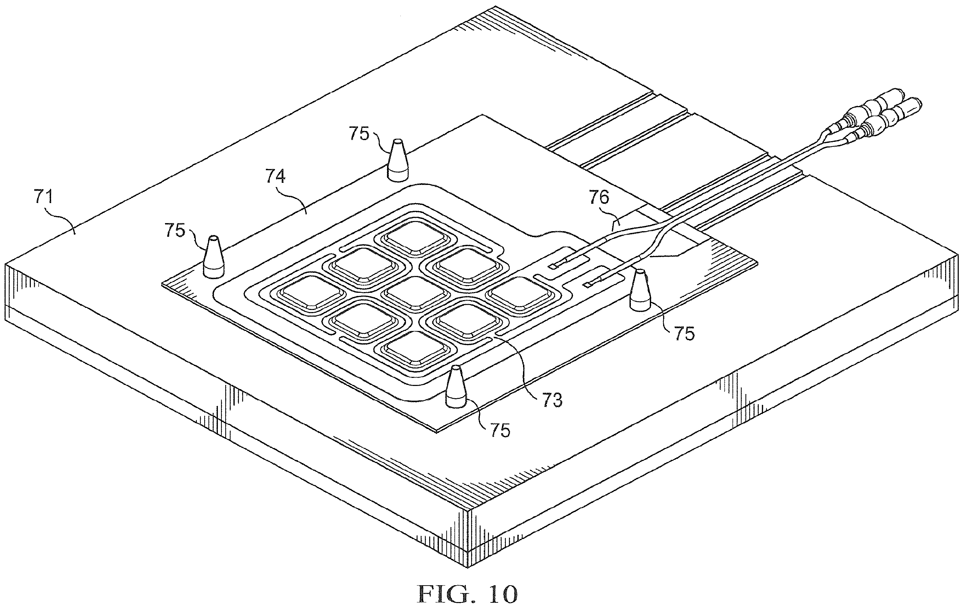

As illustrated in FIGURE 10, the flipped-over assembly is now re-seated in the receiving tray 84, preferably by aligning the alignment holes in first laminate 74 with the tapered alignment pins 75 of apparatus 71. Although it is not required, a vacuum may be reapplied as previously described.

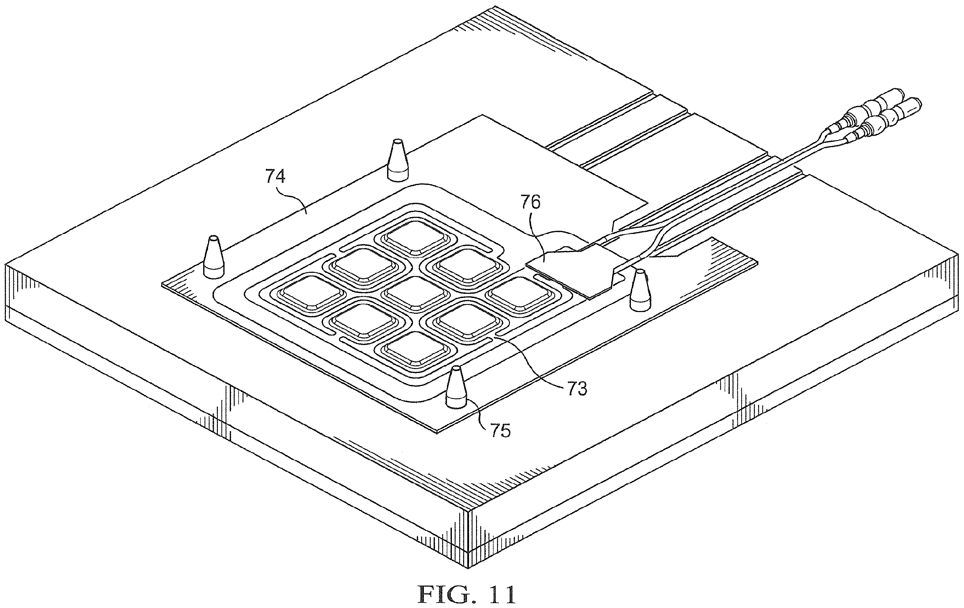

FIGURE 11 illustrates an aspect of certain cases in which first laminate 74 has a lead protector flap 76. If a liner is provided on this side of the first laminate 74, the liner portion on flap 76 may be removed. Flap 76 is folded over between the two leads. Flap 76 is positioned to cover the area at which the leads are connected to lead tabs on stamped conductive plate 73.

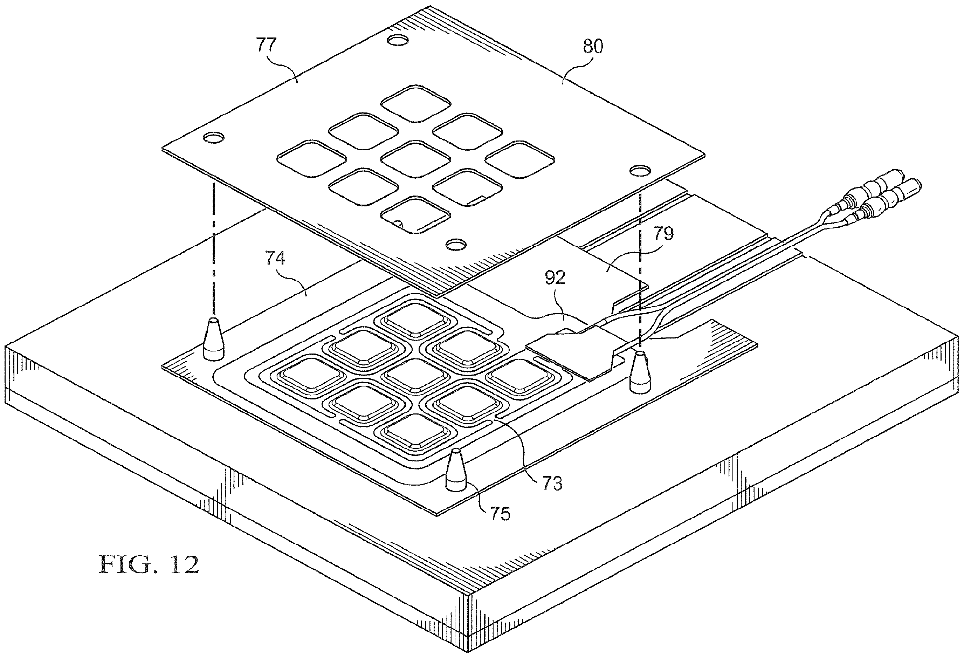

FIGURE 12 illustrates a second laminate 77 being applied to the upper surface of the assembly. If there is a paper liner covering the adhesive surface, it may be removed before applying second laminate to the assembly. Preferably, second laminate 77 has edges that are coextensive with the perimeter edges of first laminate 74 (except for electrical lead protector flap 76). Second laminate 77 preferably has a plurality of alignment holes, similar to first laminate 74 for assisting in the alignment of second laminate 77 during its application to the assembly. Second laminate 77 also has a plurality of holes configured to receive the raised protrusions as described elsewhere herein.

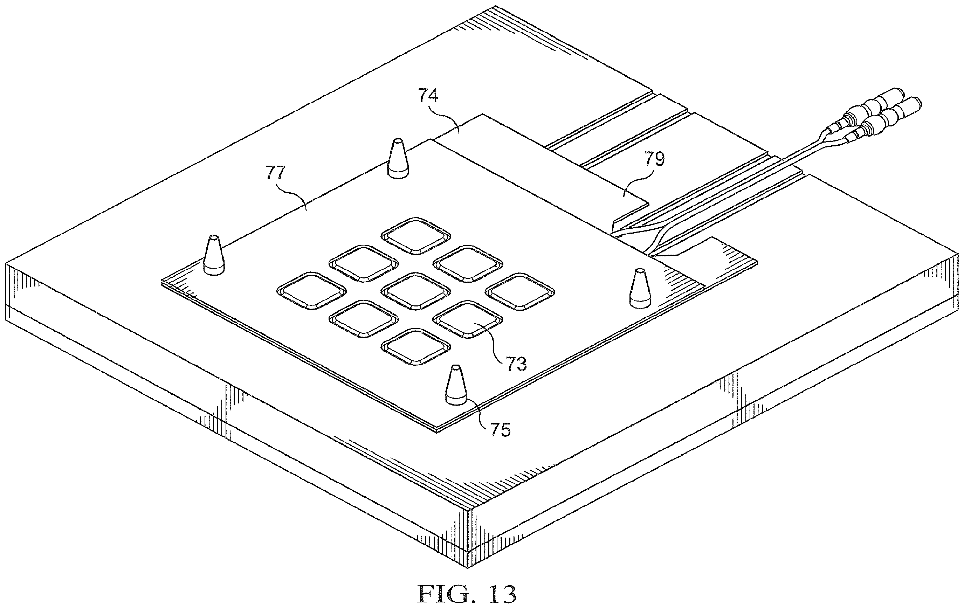

FIGURE 13 illustrates the assembly with second laminate 77 firmly affixed. Preferably second laminate 77 is affixed to the assembly as similarly described in connection with the application of first laminate 74. If the vacuum has been applied, it may now be turned off.

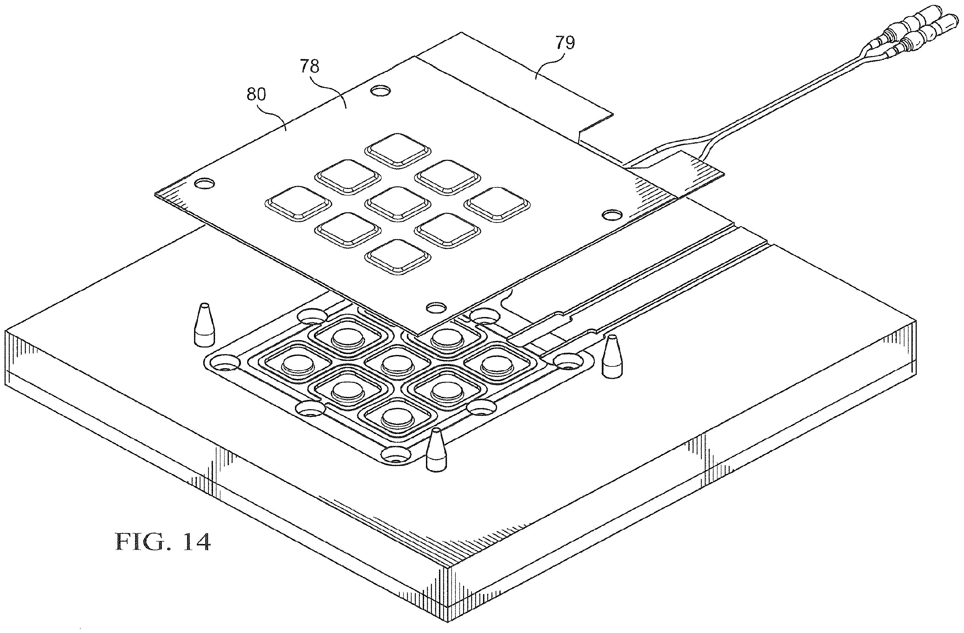

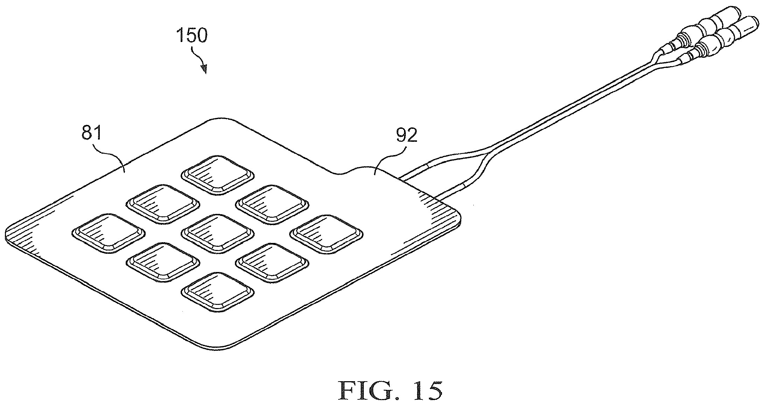

FIGURE 14 illustrates the partially-completed neurostimulation assembly removed from apparatus 71. As can be seen in FIGURES 12 and 13, first laminate 74 has a carrier portion 79. Similarly, second laminate 77 has a carrier portion 80. Carrier portions 79, 80 may be removed from their respective laminates. This leaves function portions 81 of the first and second laminates 74, 77. FIGURE 15 illustrates the completed neurostimulation device 150. Functioning laminate portions 81 of the completed device provide a lead wire extension area 92 which extends away from the electrode node array of the device.

It should be understood that the configuration of the various components of device 150, as well as the steps in the manufacturing process, may be altered.

For example, it is not critical to have the electrical lead wire extension area 92. The polarity of the leads may be reversed. Different laminate structures may be utilized to hold the two physical electrode elements in place. More or fewer vacuum holes and alignment holes may be provided. Other adjustments and modifications may be made as desired or deemed to be better for the manufacturing process, the device or its application, efficiency.

It should be understood that the various figures and their description illustrate examples of the apparatus and various aspects of the apparatus may be added, eliminated, and/or substituted for those shown. Such modifications may be made as is desired, suitable, and/or advantageous for performing the functionality described herein.

The invention is defined by the following claims.

## Claims

1. An electrode node array apparatus (60), comprising:
a first physical electrode element (31) having a first plurality of electrode nodes formed therein interconnected by a common first conductor (4); and
a second physical electrode element (32) having a second plurality of electrode nodes formed therein interconnected by a common second conductor (5);
the first and second conductors (4, 5) being arranged to interfit the first and second pluralities of electrode nodes to form an array of electrode nodes;
a first electrical lead (21) coupled to the first conductor (4); and
a second electrical lead (22) coupled to the second conductor (5), the first and second electrical leads (21, 22) adapted to be connected to a source to establish a first polarity for the first conductor (4) and a second polarity for the second conductor (5), wherein the source is capable of activating adjacent pairs of electrode nodes within the electrode node array with opposite polarities, **characterized in that** the first and second pluralities of electrode nodes are provided as first and second pluralities of raised protrusions and **in that** the first and second conductors with respective electrode elements are formable by stamping from a conductive sheet.

2. The apparatus of Claim 1, wherein the number of electrode nodes is greater than the number of physical electrode elements.

3. The apparatus of Claims 1 or 2, wherein there are two conductors, the first and the second conductors (4, 5).

4. The apparatus of Claims 1 to 3, wherein the array of electrode nodes comprises a square array having the same number of electrode nodes along each of two axes.

5. The apparatus of Claims 1 to 4, wherein the electrode nodes comprise raised protrusions stampable from a stock sheet of conductive material.

6. The apparatus of Claims 1 to 5, wherein the first and second conductors (4, 5) comprise portions of a stock sheet of conductive material and are formable simultaneously during a stamping process.

7. The apparatus of Claims 1 to 6, wherein the first conductor (4) extends along a majority of the perimeter of the device and is connected to alternating ones of the plurality of electrode nodes by a plurality of conductive bridge elements (33).

8. The apparatus of Claims 1 to 7, wherein a plurality of electrode nodes associated with the second conductor (5) is encompassed by a perimeter defined by a portion of the first conductor (4).

9. A method of making an electrode node array by stamping, comprising:
providing a conductive sheet (1);
forming a plurality of raised protrusions (3) in the conductive sheet (1) to correspond to a plurality of electrode nodes;
stamping a first conductor (4) from the conductive sheet (1), the first conductor (4) comprising a first set of the plurality of the raised protrusions interconnected by a common first conductor element;
stamping a second conductor (5) from the conductive sheet (1), the second conductor (5) comprising a second set of the plurality of raised protrusions (3) interconnected by a common second conductor element; and
interfitting the first and second conductors (4, 5), wherein the first and second sets of raised protrusions (3) are configured in an array of electrode nodes, wherein the first and second conductors are stamped from the conductive sheet in a single step.

10. The method of Claim 9, further comprising connecting a first electrical lead (21) having a first polarity to the first conductor (4) and connecting a second electrical lead (22) having a second polarity to the second conductor (5), wherein any pair of adjacent electrode nodes has opposite polarities when the first and second electrical leads (21, 22) are connected to a source.

11. The method of Claims 9 or 10, further comprising:
applying a first laminate (11) to a first side of the stamped first and second conductors (4, 5); and
applying a second laminate (12) to a second side of the stamped first and second conductors (4, 5), wherein the first and second laminates (11, 12) cooperate to hold the first conductor (4) in alignment with the second conductor (5).

12. The method of Claim 11, wherein at least one of the first and second laminates (11, 12) comprises a lead protection tab (23), and the method further comprising folding the lead protection tab over to cover at least a portion of the lead connections.

13. A combination comprising an electrode node array apparatus (60) according to claim 1 and an apparatus (71) for manufacturing the electrode node array apparatus (60), the apparatus (71) for manufacturing the electrode node array apparatus (60) comprising:
a receiving tray (84) having defined therein a plurality of recesses to correspond with a plurality of electrode nodes of the electrode node array apparatus (60); and
one or more vacuum passages (83) having a first end connectable to a vacuum source and a second end terminating at a hole at a surface of the apparatus (71), the hole operable to interact with at least a portion of an array apparatus positioned on the apparatus to hold at least a portion of the array apparatus against the apparatus (71) for manufacturing the electrode node array apparatus.

14. The combination of Claim 13, the apparatus (71) further comprising at least one suction cup (72) disposed in at least one of the plurality of recesses, the at least one suction cup (72) operable to interact with a surface of at least one of the plurality of electrode nodes of the array apparatus to hold the surface adjacent the apparatus (71).

15. The combination of Claims 13 or 14, the apparatus (71) further comprising at least one alignment pin (75) operable to receive a corresponding at least one alignment hole of a laminate associated with the array apparatus.

## Patentansprüche

1. Elektrodenknoten-Array-Vorrichtung (60), umfassend:
ein erstes physikalisches Elektrodenelement (31) mit einer ersten Mehrzahl von darin ausgebildeten Elektrodenknoten, die durch einen gemeinsamen ersten Leiter (4) miteinander verbunden sind; und
ein zweites physikalisches Elektrodenelement (32) mit einer zweiten Mehrzahl von darin ausgebildeten Elektrodenknoten, die durch einen gemeinsamen zweiten Leiter (5) miteinander verbunden sind;
wobei der erste und der zweite Leiter (4, 5) angeordnet sind, um die erste und die zweite Mehrzahl von Elektrodenknoten zusammenzufügen, um ein Array aus Elektrodenknoten zu bilden;
eine erste elektrische Leitung (21), die mit dem ersten Leiter (4) gekoppelt ist; und
eine zweite elektrische Leitung (22), die mit dem zweiten Leiter (5) gekoppelt ist, wobei die erste und die zweite elektrische Leitung (21, 22) ausgebildet sind, um mit einer Quelle verbunden zu werden, um eine erste Polarität für den ersten Leiter (4) und eine zweite Polarität für den zweiten Leiter (5) herzustellen, wobei die Quelle in der Lage ist, um benachbarte Paare von Elektrodenknoten in dem Elektrodenknoten-Array mit entgegengesetzten Polaritäten zu aktivieren, **dadurch gekennzeichnet, dass** die erste und die zweite Polarität der Elektrodenknoten als eine erste und eine zweite Polarität von erhobenen Vorsprüngen vorgesehen sind, und dass der erste und der zweite Leiter mit jeweiligen Elektrodenelementen durch Ausstanzen aus einem leitfähigen Bogen gebildet werden können.

2. Vorrichtung nach Anspruch 1, wobei die Anzahl der Elektrodenknoten größer ist als die Anzahl der physikalischen Elektrodenelemente.

3. Vorrichtung nach Ansprüchen 1 oder 2, wobei es zwei Leiter gibt, der erste und der zweite Leiter (4, 5).

4. Vorrichtung nach Ansprüchen 1 bis 3, wobei das Array aus Elektrodenknoten ein quadratisches Array mit der gleichen Anzahl von Elektrodenknoten entlang jeder von zwei Achsen umfasst.

5. Vorrichtung nach Ansprüchen 1 bis 4, wobei die Elektrodenknoten erhobene Vorsprünge aufweisen, die aus einem Ausgangsbogen aus leitfähigem Material gestanzt werden können.

6. Vorrichtung nach Ansprüchen 1 bis 5, wobei der erste und der zweite Leiter (4, 5) Bereiche eines Ausgangsbogens aus leitfähigem Material aufweisen und gleichzeitig während eines Stanzvorgangs gebildet werden können.

7. Vorrichtung nach Ansprüchen 1 bis 6, wobei sich der erste Leiter (4) entlang eines Großteils des Umfangs der Vorrichtung erstreckt und mit abwechselnden Knoten der Mehrzahl von Elektrodenknoten durch eine Mehrzahl von leitfähigen Brückenelementen (33) verbunden ist.

8. Vorrichtung nach Ansprüchen 1 bis 7, wobei eine Mehrzahl von Elektrodenknoten, die dem zweiten Leiter (5) zugeordnet sind, von einem Umfang umgeben ist, der durch einen Bereich des ersten Leiters (4) gebildet ist.

9. Verfahren zum Herstellen eines Elektrodenknoten-Arrays durch Ausstanzen, mit:
Bereitstellen eines leitfähigen Bogens (1);
Ausbilden einer Mehrzahl von erhobenen Vorsprüngen (3) in dem leitfähigen Bogen (1), um einer Mehrzahl von Elektrodenknoten zu entsprechen;
Ausstanzen eines ersten Leiters (4) aus dem leitfähigen Bogen (1), wobei der erste Leiter (4) einen ersten Satz der Mehrzahl der erhobenen Vorsprünge umfasst, die durch ein gemeinsames erstes Leiterelement miteinander verbunden sind;
Stanzen eines zweiten Leiters (5) aus dem leitfähigen Bogen (1), wobei der zweite Leiter (5) einen zweiten Satz der Mehrzahl der erhobenen Vorsprünge (3) aufweist, die durch ein gemeinsames zweites Leiterelement miteinander verbunden sind; und
Zusammenfügen des ersten und des zweiten Leiters (4, 5), wobei der erste und der zweite Satz von erhobenen Vorsprüngen (3) zu einem Array von Elektrodenknoten konfiguriert sind, wobei der erste und der zweite Leiter in einem einzigen Schritt aus dem leitfähigen Bogen ausgestanzt werden.

10. Verfahren nach Anspruch 9, das außerdem das Verbinden einer ersten elektrischen Leitung (21), die eine erste Polarität hat, mit dem ersten Leiter (4) und das Verbinden einer zweiten elektrischen Leitung (22), die eine zweite Polarität hat, mit dem zweiten Leiter (5) umfasst, wobei jedes Paar von benachbarten Elektrodenknoten entgegengesetzte Polaritäten hat, wenn die erste und die zweite elektrische Leitung (21, 22) mit einer Quelle verbunden sind.

11. Verfahren nach Ansprüchen 9 oder 10, außerdem mit:
Aufbringen eines ersten Laminats (11) auf eine erste Seite des ausgestanzten ersten und zweiten Leiters (4, 5); und
Aufbringen eines zweiten Laminats (12) auf eine zweite Seite des ausgestanzten ersten und zweiten Leiters (4, 5), wobei das erste und das zweite Laminat (11, 12) zusammenwirken, um den ersten Leiter (4) in Ausrichtung mit dem zweiten Leiter (5) zu halten.

12. Verfahren nach Anspruch 11, wobei mindestens eines des ersten und des zweiten Laminats (11, 12) eine Leitungsschutzlasche (23) aufweist, und wobei das Verfahren ferner das Umfalten der Leitungsschutzlasche umfasst, um mindestens einen Bereich der Leitungsverbindungen abzudecken.

13. Kombination mit einer Elektrodenknoten-Array-Vorrichtung (60) nach Anspruch 1 und einer Vorrichtung (71) zum Herstellen der Elektrodenknoten-Array-Vorrichtung (60), wobei die Vorrichtung (71) zum Herstellen der Elektrodenknoten-Array-Vorrichtung (60) umfasst:
eine Aufnahmeschale (84), in der eine Mehrzahl von Ausnehmungen definiert ist, um einer Mehrzahl von Elektrodenknoten der Elektrodenknoten-Array-Vorrichtung (60) zu entsprechen; und
ein oder mehrere Vakuumkanäle (83) mit einem ersten Ende, das mit einer Vakuumquelle verbindbar ist, und einem zweiten Ende, das an einem Loch an einer Oberfläche der Vorrichtung (71) endet, wobei das Loch dazu dient, um mit mindestens einem Bereich einer an der Vorrichtung positionierten Array-Vorrichtung in Wechselwirkung zu treten, um mindestens einen Bereich der Array-Vorrichtung gegen die Vorrichtung (71) zum Herstellen der Elektrodenknoten-Array-Vorrichtung zu halten.

14. Kombination nach Anspruch 13, wobei die Vorrichtung (71) außerdem mindestens einen Saugnapf (72) aufweist, der in mindestens einer der Mehrzahl von Ausnehmungen angeordnet ist, wobei der mindestens eine Saugnapf (72) dazu dient, um mit einer Oberfläche von mindestens einem der Mehrzahl von Elektrodenknoten der Array-Vorrichtung in Wechselwirkung zu treten, um die Oberfläche benachbart zu der Vorrichtung (71) zu halten.

15. Kombination nach Ansprüchen 13 oder 14, wobei die Vorrichtung (71) außerdem mindestens einen Ausrichtungsstift (75) aufweist, der dazu dient, um in einem entsprechenden mindestens einem Ausrichtungsloch eines Laminats aufgenommen zu werden, das der Array-Vorrichtung zugeordnet ist.

## Revendications

1. Appareil formant réseau de noeuds d'électrodes (60) comprenant :
un premier élément formant électrode physique (31) comportant une première pluralité de noeuds d'électrodes formés dans celui-ci, interconnectés par un premier conducteur commun (4) ; et
un deuxième élément formant électrode physique (32) comportant une deuxième pluralité de noeuds d'électrodes formés dans celui-ci, interconnectés par un deuxième conducteur commun (5) ;
les premier et deuxième conducteurs (4, 5) étant agencés pour assembler entre elles les première et deuxième pluralités de noeuds d'électrodes afin de former un réseau de noeuds d'électrodes ;
un premier fil électrique (21) couplé au premier conducteur (4) ; et
un deuxième fil électrique (22) couplé au deuxième conducteur (5), les premier et deuxième fils électriques (21, 22) étant adaptés pour être connectés à une source pour établir une première polarité pour le premier conducteur (4) et une deuxième polarité pour le deuxième conducteur (5), dans lequel la source est capable d'activer des paires voisines de noeuds d'électrodes dans le réseau de noeuds d'électrodes avec des polarités opposées, **caractérisé en ce que** les première et deuxième pluralités de noeuds d'électrodes sont fournies sous la forme de première et deuxième pluralités de protubérances relevées et **en ce que** les premier et deuxième conducteurs avec des éléments formant électrodes respectifs peuvent être formés par estampage d'une feuille conductrice.

2. Appareil selon la revendication 1, dans lequel le nombre de noeuds d'électrodes est supérieur au nombre d'éléments formant électrodes physiques.

3. Appareil selon la revendication 1 ou 2, dans lequel il y a deux conducteurs, le premier et le deuxième conducteur (4, 5).

4. Appareil selon les revendications 1 à 3, dans lequel le réseau de noeuds d'électrodes comprend un réseau carré ayant le même nombre de noeuds d'électrodes le long de chacun de deux axes.

5. Appareil selon les revendications 1 à 4, dans lequel les noeuds d'électrodes comprennent des protubérances relevées pouvant être estampées à partir d'une feuille de matériau conducteur courante.

6. Appareil selon les revendications 1 à 5, dans lequel les premier et deuxième conducteurs (4, 5) comprennent des parties d'une feuille de matériau conducteur courante et peuvent être formés simultanément au cours d'un processus d'estampage.

7. Appareil selon les revendications 1 à 6, dans lequel le premier conducteur (4) s'étend le long d'une majorité du périmètre du dispositif et est connecté à des noeuds alternés de la pluralité de noeuds d'électrodes au moyen d'une pluralité d'éléments de jonction conducteurs (33).

8. Appareil selon les revendications 1 à 7, dans lequel une pluralité de noeuds d'électrodes associée au deuxième conducteur (5) est délimitée par un périmètre défini par une partie du premier conducteur (4).

9. Procédé de fabrication d'un réseau de noeuds d'électrodes par estampage, comprenant les étapes suivantes :
fournir une feuille conductrice (1) ;
former une pluralité de protubérances relevées (3) dans la feuille conductrice (1) destinées à correspondre avec une pluralité de noeuds d'électrodes ;
estamper un premier conducteur (4) dans la feuille conductrice (1), le premier conducteur (4) comprenant un premier ensemble de la pluralité de protubérances relevées interconnectées par un premier élément conducteur commun ;
estamper un deuxième conducteur (5) dans la feuille conductrice (1), le deuxième conducteur (5) comprenant un deuxième ensemble de la pluralité de protubérances relevées (3) interconnectées par un deuxième élément conducteur commun ; et
assembler entre eux les premier et deuxième conducteurs (4, 5), dans lequel les premier et deuxième ensembles de protubérances relevées (3) sont configurés sous la forme d'un réseau de noeuds d'électrodes, dans lequel les premier et deuxième conducteurs sont estampés à partir de la feuille conductrice en une seule étape.

10. Procédé selon la revendication 9, comprenant en outre l'opération consistant à connecter un premier fil électrique (21) ayant une première polarité au premier conducteur (4) et à connecter un deuxième fil électrique (22) ayant une deuxième polarité au deuxième conducteur (5), dans lequel toute paire de noeuds d'électrodes voisins a des polarités opposées quand les premier et deuxième fils électriques (21, 22) sont connectés à une source.

11. Procédé selon la revendication 9 ou 10, comprenant en outre les étapes suivantes :
appliquer un premier stratifié (11) sur un premier côté des premier et deuxième conducteurs estampés (4, 5) ; et
appliquer un deuxième stratifié (12) sur un deuxième côté des premier et deuxième conducteurs estampés (4, 5), dans lequel les premier et deuxième stratifiés (11, 12) coopèrent pour maintenir le premier conducteur (4) dans l'alignement du deuxième conducteur (5).

12. Procédé selon la revendication 11, dans lequel au moins l'un des premier et deuxième stratifiés (11, 12) comprend une patte de protection de fils (23), et le procédé comprenant en outre l'opération consistant à replier la patte de protection de fils afin de couvrir au moins une partie des connexions de fils.

13. Combinaison comprenant un appareil formant réseau de noeuds d'électrodes (60) selon la revendication 1 et un appareil (71) permettant de fabriquer l'appareil formant réseau de noeuds d'électrodes (60), l'appareil (71) permettant de fabriquer l'appareil formant réseau de noeuds d'électrodes (60) comprenant :
un plateau récepteur (84) dans lequel est définie une pluralité d'évidements destinés à correspondre avec une pluralité de noeuds d'électrodes de l'appareil formant réseau de noeuds d'électrodes (60); et
un ou plusieurs passages à dépression (83) ayant une première extrémité pouvant être connectée à une source de vide et une deuxième extrémité se terminant au niveau d'un trou dans une surface de l'appareil (71), le trou pouvant être employé pour interagir avec au moins une partie d'un appareil formant réseau positionné sur l'appareil pour maintenir au moins une partie de l'appareil formant réseau contre l'appareil (71) permettant de fabriquer l'appareil formant réseau de noeuds d'électrodes.

14. Combinaison selon la revendication 13, dans laquelle l'appareil (71) comprend en outre au moins une ventouse (72) placée dans au moins l'un des évidements, ladite au moins une ventouse (72) pouvant être employée pour interagir avec une surface d'au moins l'un des noeuds d'électrodes de l'appareil formant réseau pour maintenir la surface adjacente à l'appareil (71).

15. Combinaison selon la revendication 13 ou 14, dans laquelle l'appareil (71) comprend en outre au moins un ergot de positionnement (75) destiné à recevoir au moins un trou d'alignement correspondant d'un stratifié associé à l'appareil formant réseau.
